# EUROPEAN PATENT APPLICATION

(11) **EP 4 574 073 A1**
(43) Date of publication of application: **25.06.2025**
(21) Application number: 24220858.5
(22) Date of filing: 18.12.2024
(51) Int. Cl.: A61B 18/14, A61B 17/00, A61M 25/00

(54) **MEDICAL PROBE WITH SLITTED TUBE AND ELECTRODES**

(30) Priority: 19.12.2023 US 202363612263 P; 02.12.2024 US 202418965139
(71) Applicant: Biosense Webster (Israel) Ltd., 2066717 Yokneam (IL)
(72) Inventor: SUAREZ, Paul, Irvine, 92618 (US); HIGHSMITH, Debby, Irvine, 92618 (US); STANLEY, Mark, Irvine, 92618 (US); RORICK, Kevin, Irvine, 92618 (US); CHAUDHRY, Zahir, Irvine, 92618 (US)
(74) Representative: Carpmaels & Ransford LLP

(57) **Abstract**

The disclosed technology includes a medical probe. The medical probe includes an elongated body, a flexible circuit and one or more electrodes. The elongated body extends along a longitudinal axis from a proximal end to a distal end, defines a lumen, and includes a tube and outer wall jacket. The tube extends along the longitudinal axis and includes slits that permit the elongated body to deflect relative to the longitudinal axis. The outer wall jacket surrounds the tube. The flexible circuit is disposed on the outer wall jacket. The one or more electrodes are disposed on the flexible circuit and are designed to be placed in contact with tissue of an organ.

## Description

### CROSS-REFERENCE TO RELATED APPLICATIONS

The application claims benefit of priority to prior filed U.S. Provisional Patent Application No. 63/612,263 filed December 19, 2023 (Attorney Docket No.: BIO6921USPSP1 - 253757.000447), which is hereby incorporated by reference as if set forth in full herein.

### FIELD

The present technology relates generally to medical devices, and in particular medical probes with electrodes, and further relates to, but not exclusively, medical probes suitable for use to map and/or ablate tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Typically, catheters are formed from a large number of components that require many complex assembly steps that are labor and/or time expensive. Accordingly, there is a need for a simplified medical probe design that addresses these problems.

### SUMMARY

There is provided, in accordance with the disclosed technology, a medical probe comprising an elongated body and one or more electrodes. The elongated body extends along a longitudinal axis from a proximal end to a distal end and defines a lumen. The elongated body comprises a tube, an outer wall jacket, and a flexible circuit. The tube extends along the longitudinal axis and comprises a plurality of slits formed therein. The slits permit the elongated body to deflect relative to the longitudinal axis. The outer wall jacket surrounding the tube. The flexible circuit is disposed on the outer wall jacket. The one or more electrodes are disposed on the flexible circuit and are configured to be placed in contact with tissue of an organ.

There is further provided, in accordance with the disclosed technology, a method of forming a medical probe. The method comprises the step of laser cutting a tube and to a predetermined length. The method comprises the step of laser cutting a plurality of slits in the tube, the slits permitting the tube to deflect relative to a longitudinal axis. The method comprises the step of covering an outer surface of the tube with an outer wall jacket. The method comprises the step of wrapping a flexible circuit at least partially around the outer wall jacket, the flexible circuit comprising one or more electrodes. The method comprises the step of heating a thermoplastic material over the probe flexible circuit and reflowing the thermoplastic material. The method comprises the step of laser drilling an aperture in the thermoplastic material to expose a surface of the one or more electrodes.

### BRIEF DESCRIPTION OF THE DRAWINGS

FIG. 1 is a schematic pictorial illustration of a medical system including a medical device that includes a medical probe with a distal tip with electrodes and coils, in accordance with the disclosed technology;
FIG. 2 is a schematic pictorial illustration showing a top view of a medical probe with the distal tip, in accordance with the disclosed technology;
FIG. 3 is a schematic pictorial illustration showing a perspective view of a portion of the medical probe, with the distal tip inserted into a vessel of the heart, in accordance with the disclosed technology;
FIG. 4 is a schematic pictorial illustration showing a cross-sectional view taken along line 4-4 in FIG. 3, in accordance with the disclosed technology;
FIG. 5 is a schematic pictorial illustration showing a cross-sectional view, similar to that of FIG. 4, with a slightly modified configuration, in accordance with the disclosed technology;
FIG. 6 is a schematic pictorial illustration showing a cross-sectional view taken along line 6-6 in FIG. 5, in accordance with the disclosed technology;
FIG. 7 is a schematic pictorial illustration showing the distal tip being bent in a first direction and a second direction, in accordance with the disclosed technology;
FIG. 8 is a schematic pictorial illustration showing various exemplary slit designs, in accordance with the disclosed technology;
FIG. 9A is a schematic pictorial illustration showing a flexible circuit in an unfolded configuration, in accordance with the disclosed technology;
FIG. 9B is a schematic pictorial illustration showing the flexible circuit of FIG. 9A in a folded configuration, in accordance with the disclosed technology;
FIG. 10A is a schematic pictorial illustration showing a top view of an exemplary flexible circuit configuration with coils and electrodes, in accordance with the disclosed technology;
FIG. 10B is a schematic pictorial illustration showing a bottom view of the exemplary flexible circuit configuration with coils and electrodes of FIG. 10A, in accordance with the disclosed technology;
FIG. 10C is a schematic pictorial illustration showing a detail view of Detail A in FIG. 10B, in accordance with the disclosed technology;
FIG. 11 is a schematic pictorial illustration showing a neutral layer of the distal tip of FIG. 2, in accordance with the disclosed technology;
FIG. 12A is a schematic pictorial illustration showing an alternative flexible circuit configuration, in accordance with the disclosed technology;
FIG. 12B is a schematic pictorial illustration showing the alternative flexible circuit configuration of FIG. 12A wrapped around the distal tip of the medical probe, in accordance with the disclosed technology;
FIG. 13A is a schematic pictorial illustration showing another alternative flexible circuit configuration, in accordance with the disclosed technology;
FIG. 13B is a schematic pictorial illustration showing the alternative flexible circuit configuration of FIG. 13A wrapped around the distal tip of the medical probe, in accordance with the disclosed technology;
FIG. 14A is a schematic pictorial illustration showing another alternative flexible circuit configuration, prior to being wrapped around the distal tip of the medical probe, in accordance with the disclosed technology;
FIG. 14B is a schematic pictorial illustration showing the alternative flexible circuit configuration of FIG. 14A wrapped around the distal tip of the medical probe, in accordance with the disclosed technology;
FIG. 15A is a schematic pictorial illustration showing another alternative flexible circuit configuration, in accordance with the disclosed technology;
FIG. 15B is a schematic pictorial illustration showing another alternative flexible circuit configuration, in accordance with the disclosed technology;
FIG. 15C is a schematic pictorial illustration showing another alternative flexible circuit configuration, in accordance with the disclosed technology;
FIG. 16 is a schematic pictorial illustration showing another alternative flexible circuit configuration, in accordance with the disclosed technology;
FIG. 17 is a flow chart depicting a method of assembling a flexible circuit to the distal tip of the medical probe, in accordance with the disclosed technology;
FIG. 18 is a flow chart depicting another method of assembling a flexible circuit to the distal tip of the medical probe, in accordance with the disclosed technology;
FIG. 19 is a flow chart depicting another method of assembling a flexible circuit to the distal tip of the medical probe, in accordance with the disclosed technology;
FIG. 20 is a flow chart depicting another method of assembling a flexible circuit to the distal tip of the medical probe, in accordance with the disclosed technology;
FIG. 21 is a flow chart depicting another method of assembling a flexible circuit to the distal tip of the medical probe, in accordance with the disclosed technology;
FIG. 22 is a schematic pictorial illustration showing a first connecting feature for connecting a proximal end of an elongated tube with a handle of the medical probe, in accordance with the disclosed technology;
FIG. 23 is a schematic pictorial illustration showing a second connecting feature for connecting the proximal end of the elongated tube with the handle of the medical probe, in accordance with the disclosed technology; and
FIG. 24 is a schematic pictorial illustration showing a sectional view of the handle, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

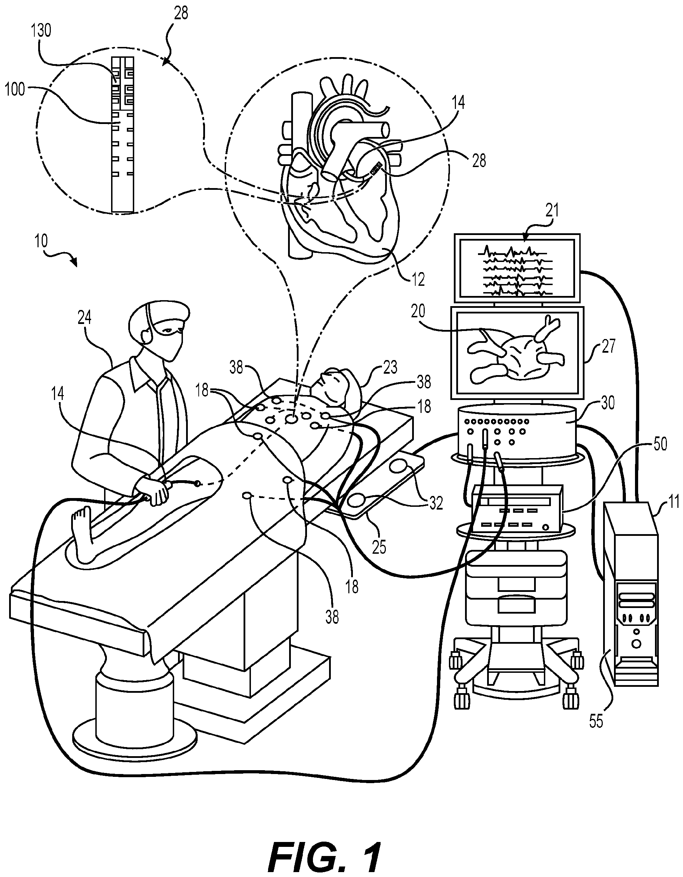

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example medical device/probe, e.g., a catheter 14 (also referred to synonymously herein as a medical probe 14), that is configured for sensing IEGM is illustrated herein. Physician 24 brings a distal tip of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 configured to sense the IEGM signals. In examples described herein, electrodes 26 can be configured to deliver ablation energy (IRE and/or RF) to tissue in heart 12. In addition to using electrodes 26 to deliver ablation energy, the electrodes 26 can also be used to determine the location of the distal tip 28 and/or to measure a physiological property such as local surface electrical potentials at respective locations on tissue in heart 12. The electrodes 26 can be biased such that a greater portion of the electrode 26 faces outwardly from distal tip 28 such that the electrodes 26 deliver a greater amount of electrical energy outwardly away from distal tip 28 (i.e., toward the heart 12 tissue) than inwardly toward the distal tip 28.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes 26 on the inner sides of the spines), and then to the blood pool in heart 12.

Catheter 14 may additionally include a position sensor embedded in or near distal tip 28 for tracking position and orientation thereof. Optionally and preferably, position sensor is a magnetic based position sensor including three magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage direct current (DC) or alternating current (AC) pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

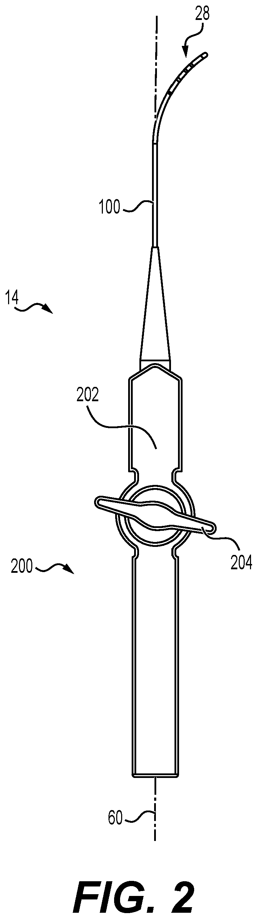

FIG. 2 is a schematic pictorial illustration showing a front view of a medical probe 14 with the distal tip 28. A medical probe 14 in accordance with the present disclosure generally includes an elongated body 100 that extends along a longitudinal axis 60 from a proximal end to a distal end and defines a lumen 104 therethrough. The elongated body 100, at proximal end thereof, is connected to a handle 200 and extends to the distal tip 28. The handle 200 includes a handle housing 202 and a pull wire control knob 204, which is discussed in greater detail below.

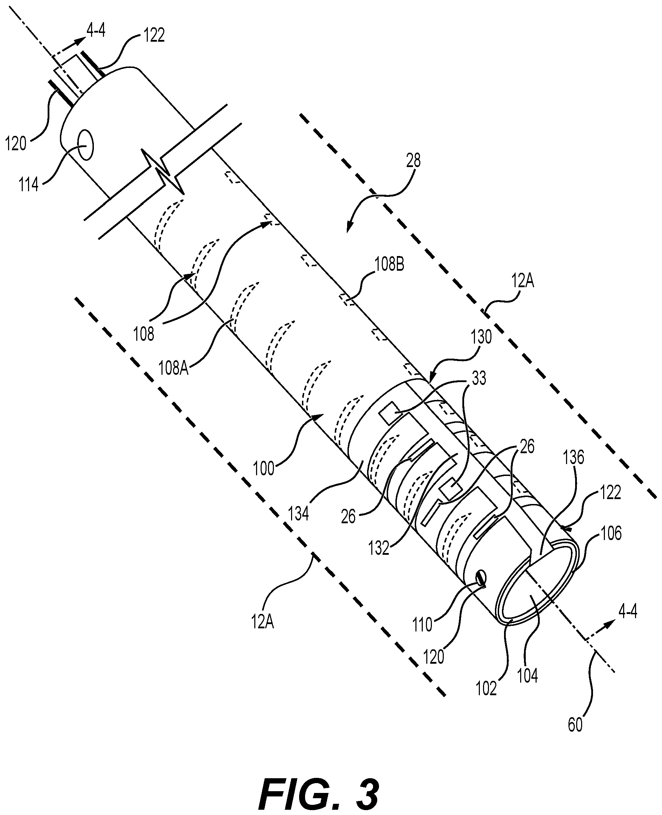

FIG. 3 is a schematic pictorial illustration showing a perspective view of the probe body 100 of the medical probe 14, with the distal tip inserted into a vessel 12A of the heart 12. As exemplarily depicted, the elongated body 100 includes an elongated tube 102 (that defines the lumen 104 and extends along the longitudinal axis 60) and an outer wall jacket 106 that surrounds the tube 102.

The tube 102 can be monolithic/unitary in form (i.e., a single piece of material or member) and made from a biocompatible metal material, such as stainless steel, rather than a braided mesh. In some examples, the tube 102 can be laser cut to a predetermined length. As will be appreciated by those in the art, the elongated body 100, when navigating within a patient 23, must be able to deflect to navigate the vessels it traverses. Thus, in order for the tube 102 to be able to bidirectionally deflect relative to the longitudinal axis 60, the tube 102 includes a plurality of slits 108 formed therein.

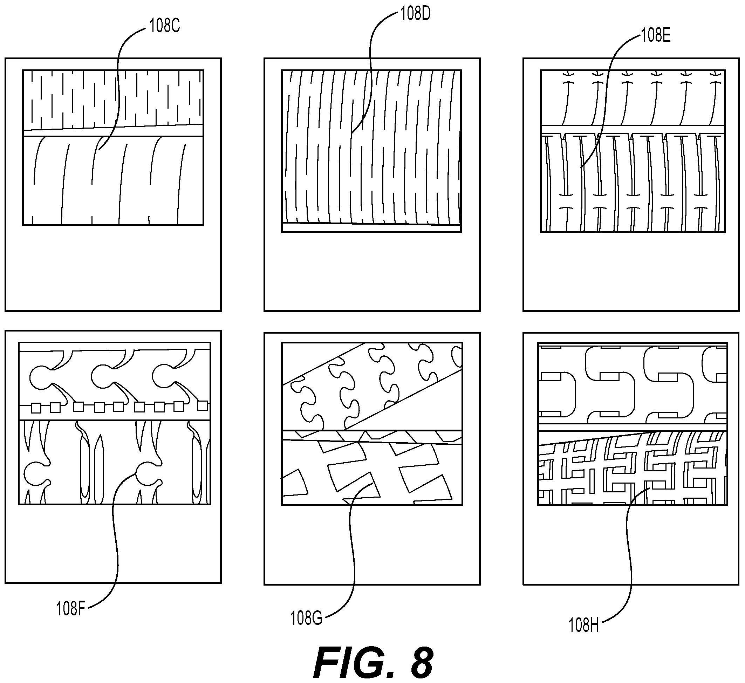

In some examples, these slits 108 can be laser cut in an approximately orthogonal direction relative to the longitudinal axis 60. However, the slits may take various forms, such as those depicted in FIG. 8. Solely by way of example, the slits may formed in a spiral shape 108C, a brickwork shape 108D, a dogbone shape 108E, a ball joint shape 108F, a dovetail puzzle shape 108G, a T-slot shape 108H, and combinations thereof to achieve the flexibility required for proper operation of the medical probe 14.

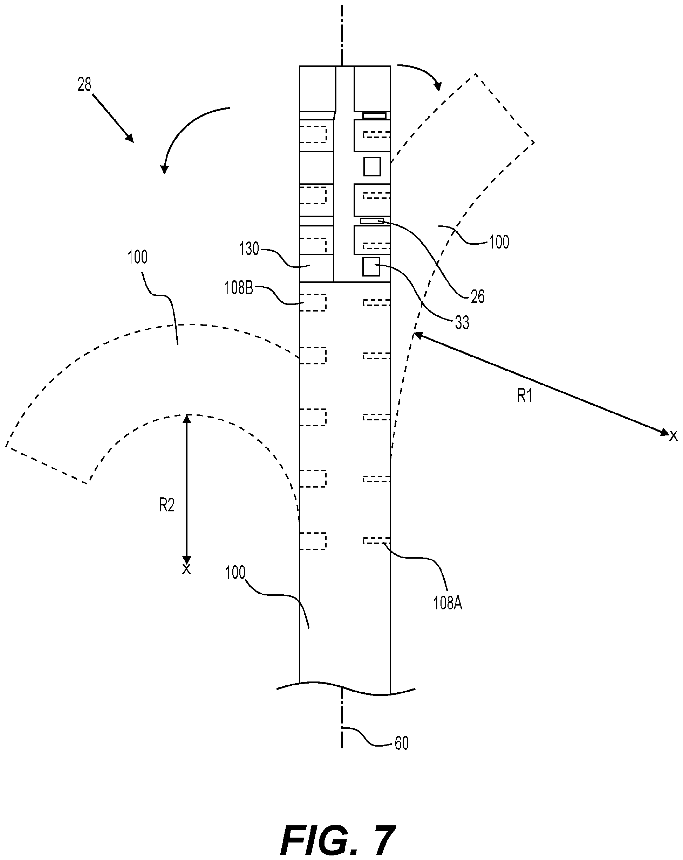

FIG. 7 is a schematic pictorial illustration showing a top view of the distal tip 28, with it being bent in two opposing directions, as denoted by the phantom lines. In the present example, and as shown in phantom lines in FIGs. 3 and 7, a plurality of first slits 108A can be formed in a first side of the tube 102 and a plurality of second slits 108B can be formed in a second side of the tube 102 that is opposite the first side. It is noted that the slits 108 are shown in phantom lines in FIGs. 3 and 7 due to the outer wall jacket 106 covering them. The slits 108 can be formed with different slit profiles that result in asymmetric deflection, depending upon the direction the tube 102 is bent. As exemplarily depicted, and with specific reference to FIG. 7, the first slits 108A can have a first width, and the second slits 108B can have a second width that is greater than the first width.

Consequently, when the tube 102 is deflected to its maximum extent (with a predetermined maximum applied force that does not cause permanent deformation of the tube 102) in a clockwise direction (relative to FIG. 7), the elongated body 100 has a first bend radius R1 with a first radius of curvature. Conversely, when the tube 102 is deflected to its maximum extent (with a predetermined maximum applied force that does not cause permanent deformation of the tube 102) in a counterclockwise direction (relative to FIG. 7), the elongated body 100 has a second bend radius R2 with a second radius of curvature that is smaller than that of the first bend radius R1. These differing bend radii are possible due to the asymmetric design of the slits 108 in the tube 102.

The outer wall jacket 106, which covers an outer surface of the tube 102, may be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3 -hydroxyvalerate) (PHBV), poly-L-lactide, polydioxanone, polycarbonates, and polyanhydrides with the ratio of certain polymers being selected to control the degree of inflammatory response.

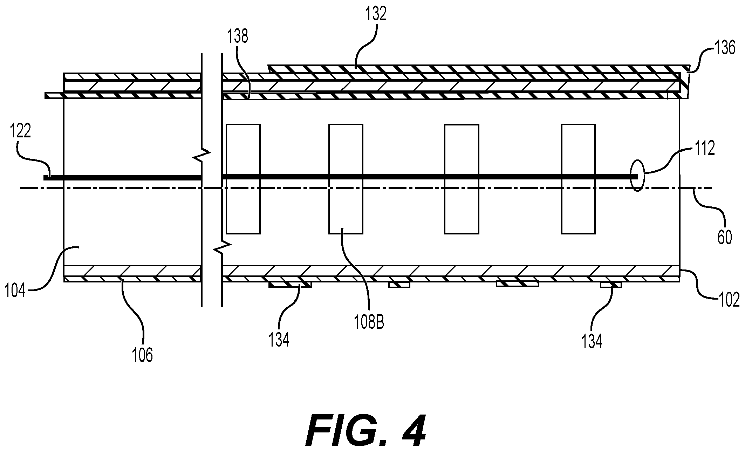

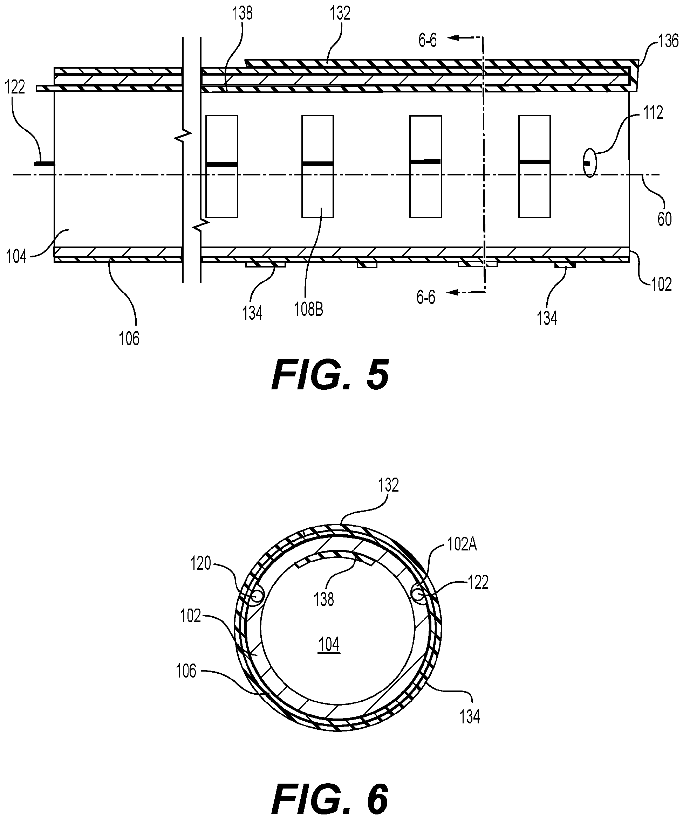

FIG. 4 is a schematic pictorial illustration showing a cross-sectional view taken along line 4-4 in FIG. 3. FIG. 5 is a schematic pictorial illustration showing a cross-sectional view, similar to that of FIG. 4, with a slightly modified configuration of pull wire routing. FIG. 6 is a schematic pictorial illustration showing a cross-sectional view taken along line 6-6 in FIG. 5.

Making specific reference to FIGs. 3-6, the medical probe 14 includes one or more pull wires 120, 122 that extend along the longitudinal axis 60 from the distal end to the proximal end of the elongated body 100. In the presently described example, there are two pull wires: a first pull wire 120 and a second pull wire 122. Of course, it will be appreciated that other designs may include, for example, one, three, four, or more pull wire configurations without departing from the spirt and scope of the present disclosure.

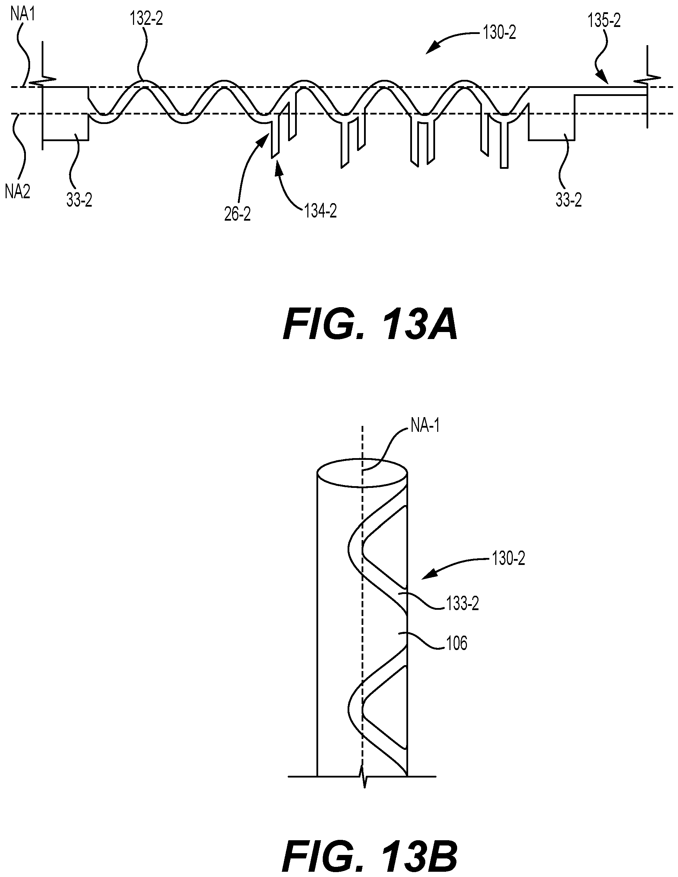

The pull wires 120, 122 are connected to the elongated body 100 in such a way so as to deflect the elongated body 100 upon movement of the respective pull wire 120, 122 via operation of the control knob 204. As shown in FIG. 13, each pull wire 120, 122 is routed into the handle 200 and connects to the control knob 204 such that operation of the control knob 204 causes the pull wires 120, 122 to respectively cause the elongated body 100 to deflect in different directions. By way of example, and referring to FIG. 7, operation of the control knob 204 in a first direction causes the first guide wire 120 to be wrapped therearound, causing the elongated body 100 to rotate in a clockwise direction. With continued reference to FIG. 7, operation of the control knob 204 in a second, opposite direction causes the second guide wire 122 to be wrapped therearound, causing the elongated body 100 to rotate in a counterclockwise direction.

To secure the first pull wire 120 and the second pull wire 122 respectively to the elongated body 100, a first hole 110 can be formed on one side of the elongated body 100 (at a distal end thereof), and a second hole 112 can be formed on another side (e.g., an opposing side at the distal end) of the elongated body 100, with the first pull wire 120 being routed through the first hole 110 and the second pull wire 122 being routed through the second hole 112. The pull wires 120, 122 can be attached to the elongated tube 102 in the openings 110, 112 via, for example, a welding process.

FIG. 4 depicts one exemplary pull wire routing configuration, whereas FIGs. 5 and 6 depict another pull wire routing configuration in accordance with the presently disclosed technology. As shown in FIG. 4, the pull wires 120, 122 can be routed within the lumen 104 from the distal end to the handle 200 along the longitudinal axis 60. In other examples, and as shown in FIGs. 5 and 6, the pull wires 120, 122 can be routed through slots 102A defined in the elongated tube 102, such that the pull wires 120, 122 are each sandwiched between the elongated tube 102 and the outer wall jacket 106 (see FIG. 6). These slots 102A can be formed in the tube 102 by laser cutting along a predetermined length of the elongated tube 102. Then, prior to jacketing of the tube 102, the pull wires 120, 122 are positioned in the slots 102A.

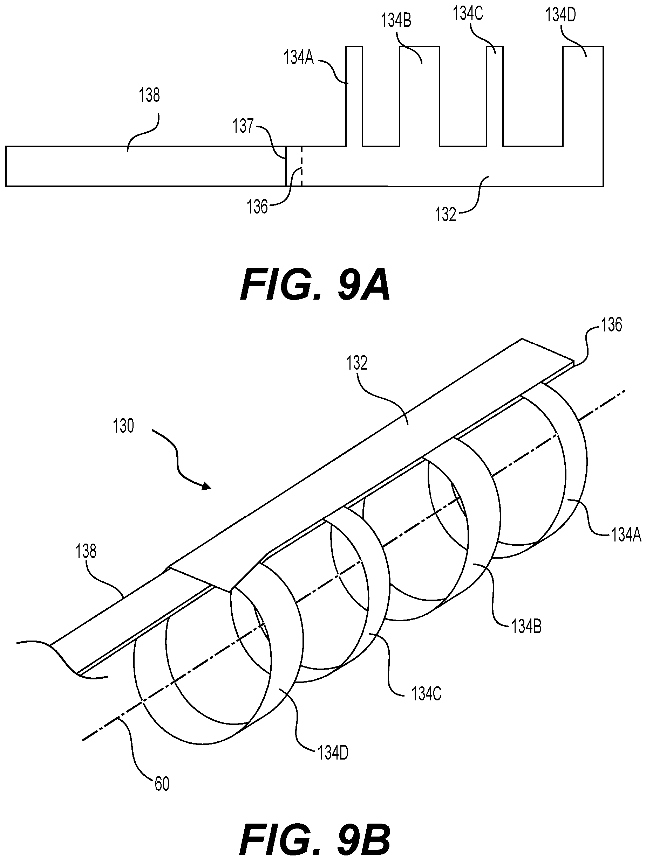

FIG. 9A is a schematic pictorial illustration showing a flexible circuit 130 in an unfolded configuration. FIG. 9B is a schematic pictorial illustration showing the flexible circuit 130 in a folded configuration, in accordance with the disclosed technology.

Referring primarily to FIGs. 3, 4, 9A, and 9B, in order to provide location-tracking, mapping, and/or ablative functionality, the distal tip 28 of the medical probe 14 is provided with a flexible circuit 130 that is disposed on that outer wall jacket 106. By way of example, the flexible circuit 130 can be a flexible printed circuit board (PCB) having one or multiple layers and printed electrical interconnections. The flexible circuit 130 can be securely connected to the distal tip 28 by heating a thermoplastic material over the probe flexible circuit 130 and reflowing the thermoplastic material. As particularly seen in FIG. 9A, the flexible circuit 130 is subdivided into a plurality of sections, namely a first section 132, one or more second sections 134, and a third section 136.

When assembled with the elongated body 100, the first section 132 extends approximately parallel to the longitudinal axis 60. As schematically shown in FIG. 7, the first section 132 says in the neutral axis of bend when the elongated body 100 is deflected.

As seen in FIGs. 3, 9A, and 9B, the second sections 134 extend from the first section 134 (approximately perpendicularly). These sections 134 are wrapped partially or entirely around an outer diameter of the outer wall jacket 106 (see the wrapping in FIG. 9B as well as FIG. 3) and are used support the various electrodes and/or coils employed, which is discussed in greater detail below. For example, there may be four second sections 134A-134D of varying or equal widths.

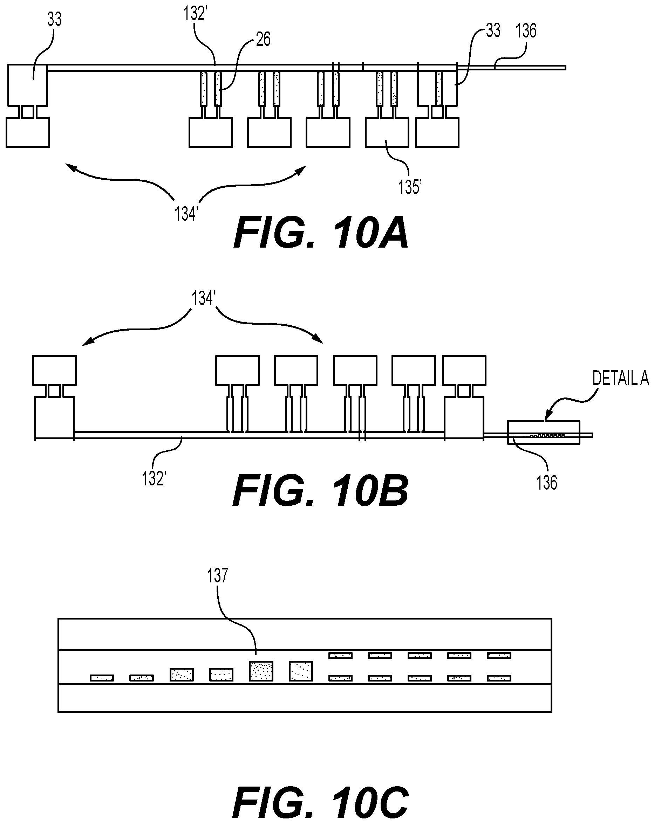

As exemplified by FIGs. 10A and 10B, another example of the first section 132' connects with any number of second sections 134' (e.g., six second sections 134') having various electrode/coil configurations (discussed in greater detail below). Moreover, the second sections 134, 134' can include tabs 135' used to aid in the assembly of the second sections 134, 134' with the outer diameter of the outer wall jacket 106.

The third section 136 extends from the first section 132 and wraps/bends around a distalmost end of the elongated body 100 such that it extends into the lumen 104, which enables electrical interconnections to be routed to a board connector 220 within the handle 200 (and connected by, for example, a zero-force socket 222). In some examples, the third section 136 can be connected to a long flexible circuit 138 via an interconnection 137 (see FIG. 10C, which shows the termination for the electrodes 26 and coils 33 for anisotropic conductive film bonding, soldering, or the like), with the long flexible circuit 138 running through the lumen 104 from near the proximal end to near the distal end and providing the connection with the board connector 220. Alternatively, the flexible circuit 130 and the long flexible circuit 138 can be configured as a single flexible circuit that extends from near the proximal end of the elongated body 100 to near the distal end of the elongated body 100.

As alluded to above, and as seen particularly in FIGs. 3 and 10A, one or more electrodes 26 are disposed on a surface of the flexible circuit 130. Specifically, the electrodes can be formed on one or more of the second sections 134 that wrap around the elongated body 100, and are designed to be placed in contact with tissue of an organ (e.g., vessel 12A of heart 12). When a thermoplastic material is heated over the flexible circuit 130 and reflowed, as mentioned above, an aperture in the thermoplastic material can be laser drilled to expose a surface of electrodes 26.

Moreover, one or more coils 33 can be disposed on the flexible circuit 130 along the longitudinal axis 60. Each coil 33 is configured to generate a current when subjected to a magnetic field, the current being indicative of a position of the respective coil 33. In some examples, each coil 33 can comprise a generally planar spiral coil formed on one of the second sections 134A-134D. The coils 33 can comprise electrical leads for conduction of current induced on each coil 33 to the patient interface unit 30. As will be appreciated, by attaching a plurality of coils 33 to the distal tip 28, it is possible to detect a position thereof. In this way, a physician 24 can more accurately determine the position of the distal tip before using it to sense anatomical signals and/or apply ablative energy to tissue in the heart 12.

As exemplified in FIGs. 3 and 10A, any number of coil 33 and electrode 26 configurations can be employed on the second sections 134. For example, some second sections 134 can include no electrodes 26 nor coils (and are primarily used to attached the flexible circuit 130 to the elongated body 100), some second sections 134 can include only electrode(s) 26, some second sections 134 can include only coil(s) 33, and some second sections 134 can include a combination of electrode(s) 26 and coil(s) 33.

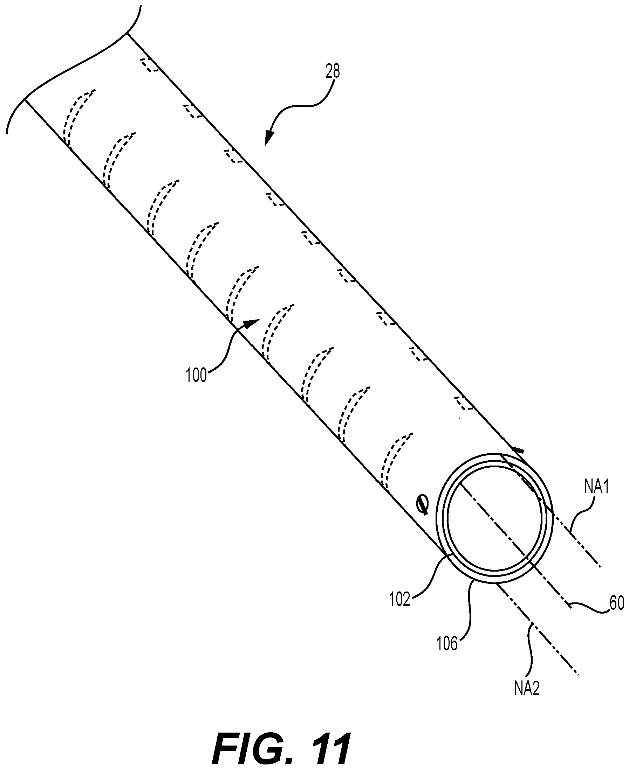

As discussed above, the tube 102 of the present disclosure includes slits 108 that give it a preferred bi-directional bending motion, where its neutral bending axis coincides with the longitudinal axis 60. When subjected to the bi-directional bending, the neutral layer of the tube 102 runs along a plane that is oriented vertically and is coplanar with the longitudinal axis. For the purposes of illustration and explanation, FIG. 11 is an exemplary illustration of the location where the neutral layer is bisects the upper portion of the elongated body 100 (illustrated and referred to herein as a first neutral axis NA1 for the purposes of explanation) and the location where the neutral layer bisects the lower portion of the elongated body 100 (illustrated and referred to herein as a second neutral axis NA2 for the purposes of explanation).

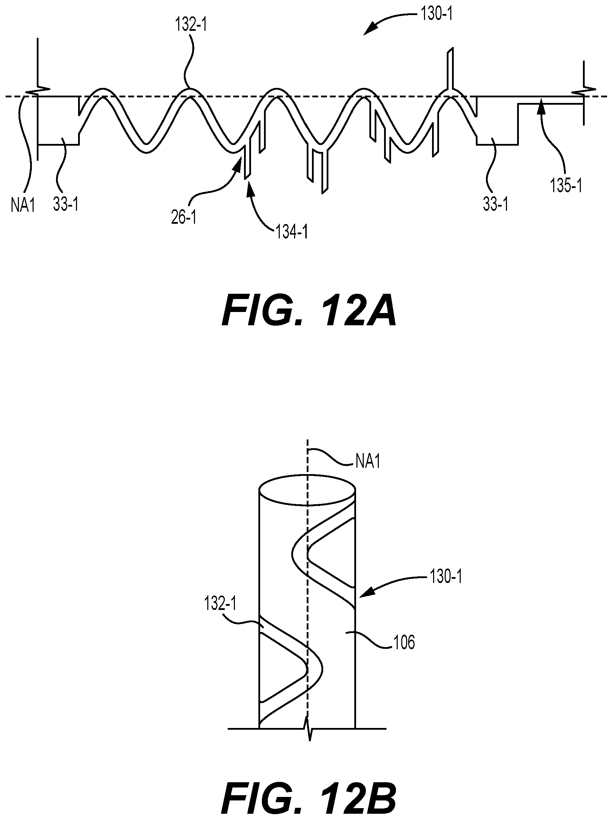

FIGs. 12A-16 depict alternative exemplary configurations of the flexible circuit 130 in accordance with the present disclosure. These examples can share features of the previously described examples, with differences noted in the below description.

As seen in FIGs. 12A-12B, the flexible circuit 130-1 includes a first section 132-1 that has a serpentine shape (in this example, its wave pattern is angled approximately 60-65 degrees relative to the first neutral axis NA1), rather than running approximately parallel to the longitudinal axis 60. The flexible circuit 130-1 also includes second sections 134-1, at least some of which electrodes 26-1 are disposed thereon, on an outer section of the serpentine shape, one or more coils 33-1, and a coil alignment marker 135-1.

In FIG. 12A, the first neutral axis NA1 is depicted to illustrate the positioning of the flexible circuit 130-1 when assembled with the tube 102 around the outer wall jacket 106. Specifically, and as best seen in FIG. 12B, the amplitude of serpentine shape is selected such that the inner edges of peaks/troughs align with (or approximately align with) the first neutral axis NA1 and each other. In other words, by virtue of the amplitude (or width) of the first section 132-1 relative to the circumference of the outer wall jacket 106, the first section 132-1 entirely wraps around the outer wall jacket 106 and the peaks and troughs of the serpentine shape align with the first neutral axis NA1 on the upper portion of the elongated body 100 (and the peaks and troughs point in opposite directions). By aligning these inner edges on the neutral layer of the elongated body 100, strain and compression on the peaks/troughs of the flexible circuit 130-1 can be reduced. It is also noted that edges of the coils 33-1 and the alignment marker 135-1 are configured relative to the first section 132-1 such that they run along the first neutral axis NA1, when assembled with the elongated body 100. With this design, proper alignment of the flexible circuit 130-1 can be better ensured.

FIGs. 13A-13B depict a similar configuration of flexible circuit 130-2 as that of the flexible circuit 130-1 depicted in FIGs. 12A-12B, but modified such that the serpentine shape (in this example, its wave pattern is angled approximately 50-55 degrees relative to the first neutral axis NA1) wraps around only a portion of the elongated body 100, rather than the entire circumference. The flexible circuit 130-2 also includes second sections 134-2, at least some of which electrodes 26-2 are disposed thereon, on an outer section of the serpentine shape, one or more coils 33-2, and a coil alignment marker 135-2.

In FIG. 13A, the first neutral axis NA1 and second neutral axis NA2 are depicted to illustrate the positioning of the flexible circuit 130-1 when assembled with the tube 102 around the outer wall jacket 106. Specifically, the amplitude of serpentine shape is selected such that the inner edges of peaks align with (or approximately align with) the first neutral axis NA1 and the inner edges of the troughs align with (or approximately align with) the second neutral axis. In other words, by virtue of the amplitude (or width) of the first section 132-2 relative to the circumference of the outer wall jacket 106, the first section 132-2 wraps around half/one side of the outer wall jacket 106 (with the exception of a small portion of the peaks/troughs) and the peaks and troughs of the serpentine shape respectively align with the first neutral axis NA1 on the upper portion of the elongated body 100 and the second neutral axis NA2 on the lower portion of the elongated body 100. By aligning these inner edges on the neutral layer of the elongated body 100, strain and compression on the peaks/troughs of the flexible circuit 130-2 can be reduced. It is also noted that edges of the coils 33-2 and the alignment marker 135-2 are configured relative to the first section 132-2 such that they run along the first neutral axis NA1, when assembled with the elongated body 100. With this design, proper alignment of the flexible circuit 130-2 can be better ensured.

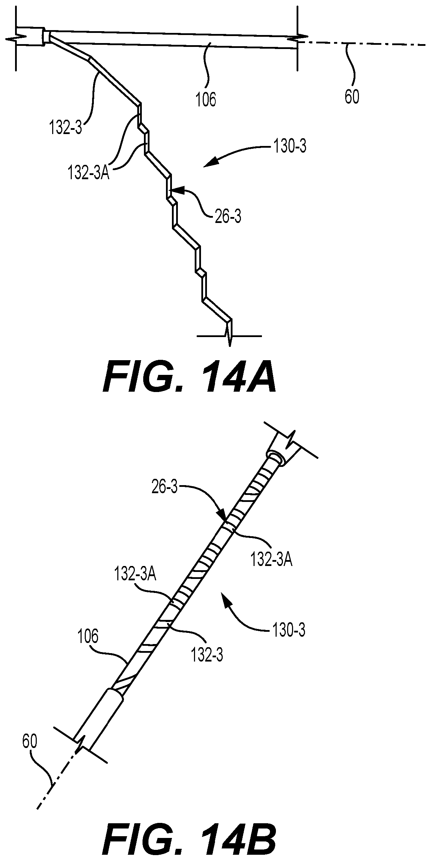

FIGs. 14A-14B depict another exemplary configuration of flexible circuit 130-3. In this example, the flexible circuit 130-3 is wrapped around the outer wall jacket 106 of the elongated body 100 in a spiraling pattern (similar to the white, red and blue stripes of a barber pole). In some examples, the electrodes can be provided along the spiraling surfaces such that they form spiral electrode surfaces when wrapped around the outer wall jacket 106. In the present example, the first section 132-3 of the flexible circuit 130-3 includes straight section 132-3 that, when wrapped around the outer wall jacket 106, are orthogonal to the longitudinal axis 60. As seen in FIG. 14B, the electrodes 26-3 can be disposed along these straight sections 132-3 such that they are orthogonal to the longitudinal axis 60 (i.e., they do not spiral along the longitudinal axis 60). These electrodes 26-3 can also be disposed on sections similar to the sections 134 depicted in FIG. 9A that protrude out from the first section, such that they extend orthogonal to the longitudinal axis 60 when wrapped around the outer wall jacket 106.

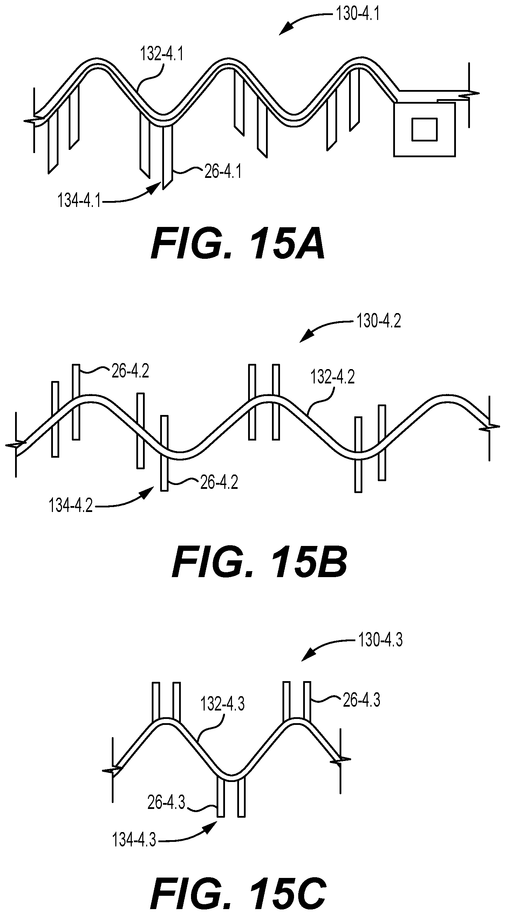

FIGs. 15A-15C depict similar examples to that of FIGs. 12A-12B and 13A-13B. In FIG. 15A, the flexible circuit 130-4.1 includes a serpentine first section 132-4.1 and a plurality of second sections 134-4.1, at least some of which support electrodes 26-4.1. In this example, the second sections 134.4.1 are configured as flaps that protrude from one side of the first section 132-4.1. In FIG. 15B, the flexible circuit 130-4.2 includes a serpentine first section 132-4.2 and a plurality of second sections 134-4.2, at least some of which support electrodes 26-4.2. In this example, the second sections 134.4.2 are configured as flaps that each protrude from both sides of the first section 132-4.2. In FIG. 15C, the flexible circuit 130-4.3 includes a serpentine first section 132-4.3 and a plurality of second sections 134-4.3, at least some of which support electrodes 26-4.3. In this example, the second sections 134.4.3 are configured as flaps that alternatingly protrude from different sides of the serpentine first section 132-4.3 such that they do not coincide or extend from with the inner curve (i.e., the concave portion) of the first section 132-4.3.

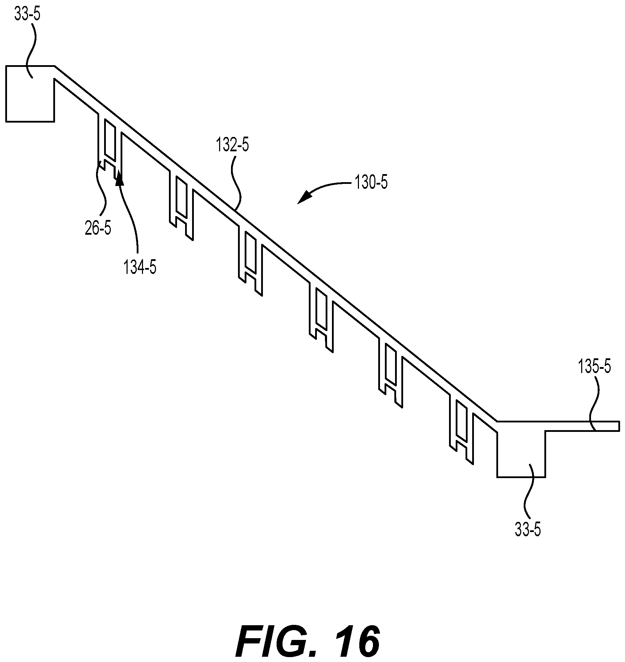

FIG. 16 depicts a similar example to that of the example of FIG. 14A-14B, which wraps around the outer wall jacket 106. The flexible circuit 130-5 includes one or more coils 33-5, a first section 132-5 that spirals around the elongated body 100 when assembled, a plurality of second sections 134-5, at least some of which having electrodes 26-5 provided thereon, and a coil alignment marker 135-5. In this example, adjacent second sections 134-5 that support the electrodes 26-5 can be physically (but not electrically) connected together to aid in support and assembly with the elongated body 100.

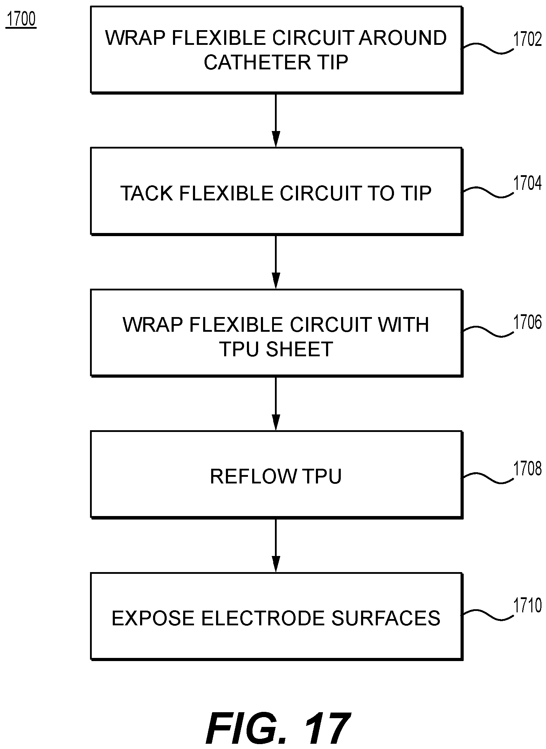

FIGs. 17-21 depict exemplary methods of assembling the previously described flexible circuits to the elongated body 100.

As seen in FIG. 17, a method 1700 of assembling a flexible circuit can include the following. A flexible circuit is wrapped 1702 around a catheter tip (e.g., the distal tip 28). The flexible circuit is tacked 1704 (i.e., at least temporarily held/maintained in position) to the catheter tip. The flexible circuit is wrapped 1706 with a biocompatible sheet, such as a polymer like TPU. The TPU is reflowed 1708, and the electrode surfaces of the flexible circuit are exposed 1710.

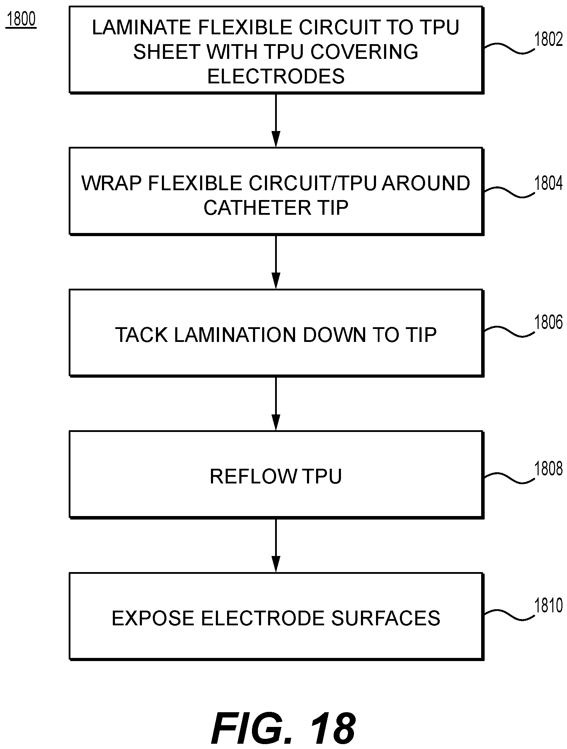

As seen in FIG. 18, another method 1800 of assembling a flexible circuit can include the following. A flexible circuit is laminated 1802 to a biocompatible sheet (e.g., TPU), with the biocompatible sheet covering electrodes disposed on the flexible circuit. The laminated flexible circuit and TPU is wrapped 1804 around a catheter tip (e.g., the distal tip 28). The laminated flexible circuit and TPU is tacked 1808 to the catheter tip. The TPU is reflowed 1808, and the electrode surfaces of the flexible circuit are exposed 1810.

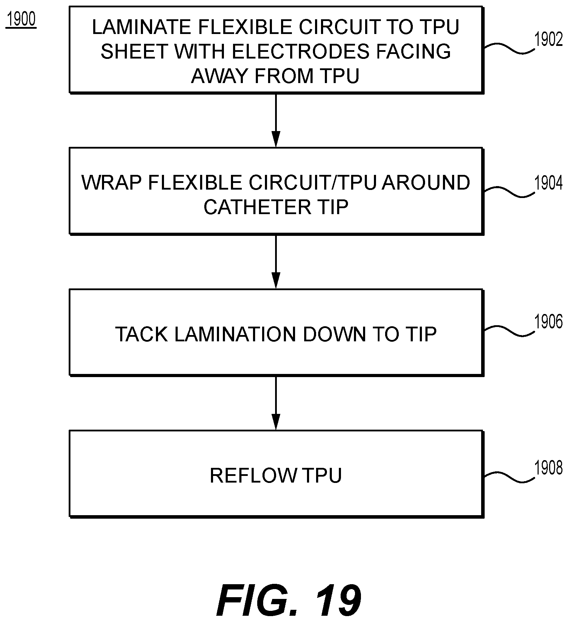

As seen in FIG. 19, another method 1900 of assembling a flexible circuit can include the following. A flexible circuit is laminated 1902 to a biocompatible sheet (e.g., TPU), with electrodes disposed on the flexible circuit facing away from the TPU. The laminated flexible circuit and TPU assembly is wrapped 1904 around a catheter tip (e.g., distal tip 28). The laminated flexible circuit and TPU is tacked 1906 to the catheter tip. The TPU is reflowed 1908. Since the electrodes were initially positioned facing away from the TPU, they are exposed in the final assembly.

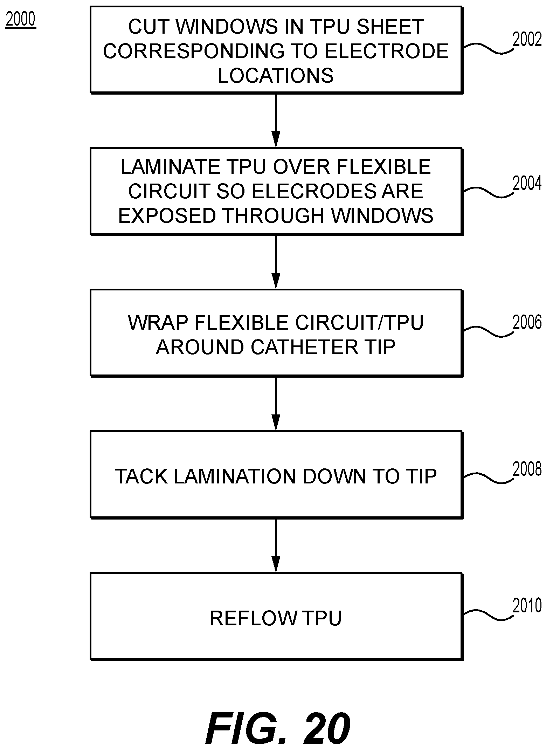

As seen in FIG. 20, another method 2000 of assembling a flexible circuit can include the following. Windows in a biocompatible sheet (e.g., TPU) are cut 2002 corresponding to electrode locations in a flexible circuit. The TPU is laminated 2004 over the flexible circuit so electrodes are exposed through the windows. The laminated flexible circuit and TPU assembly is wrapped around a catheter tip (e.g., distal tip 28).

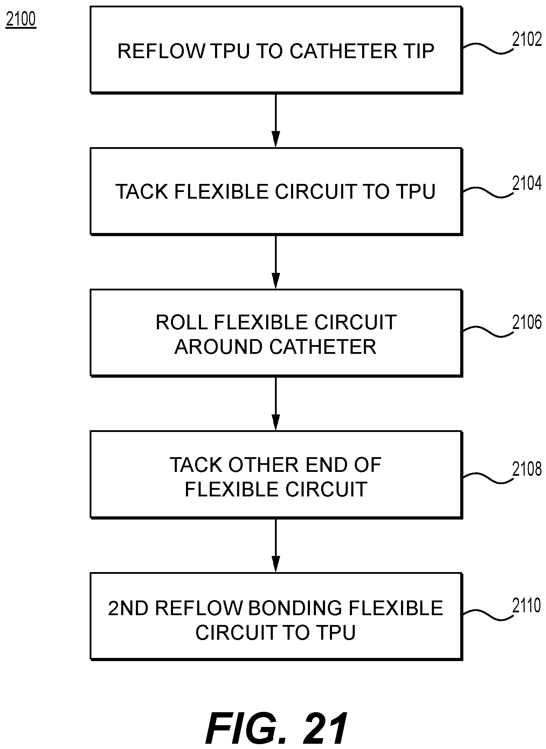

As seen in FIG. 21, another method 2100 of assembling a flexible circuit can include the following. A biocompatible sheet (e.g., TPU) is reflowed 2102 to a catheter tip (e.g. distal tip 28). One end of a flexible circuit is tacked 2104 to the TPU. The flexible circuit is rolled 2106 around the catheter tip. The other end of the flexible circuit is tacked 2108 to the TPU. The TPU is reflowed 2110 again, which bonds the flexible circuit to the TPU.

In addition to alternatively to the above-described methods, the flexible circuit can be wrapped and tacked onto the catheter tip (e.g., with adhesive) and then the catheter and flex may be overmolded by a suitable low-temperature process (e.g., liquid silicone) with appropriate features in the tooling to ensure the electrode surfaces remain exposed.

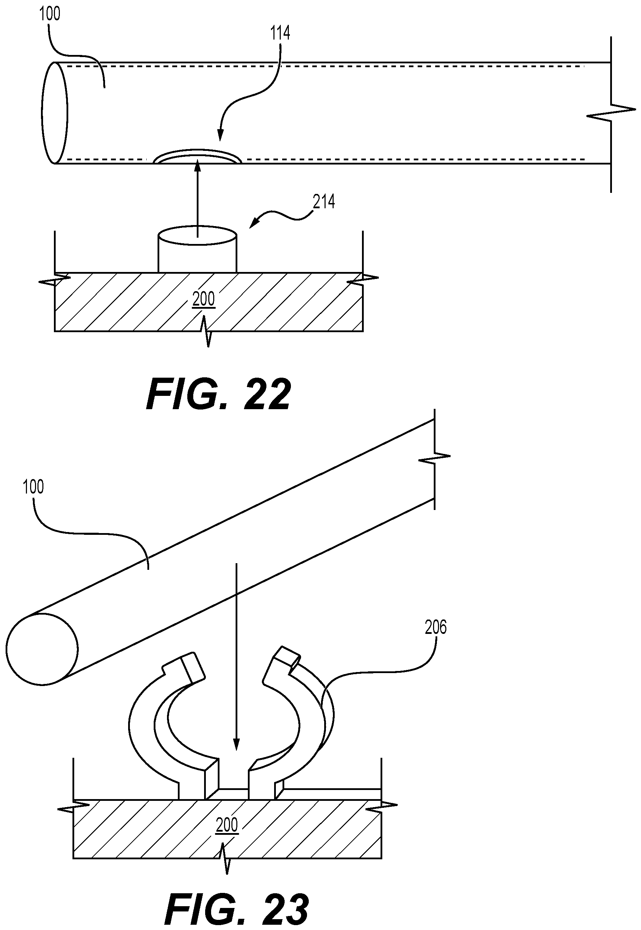

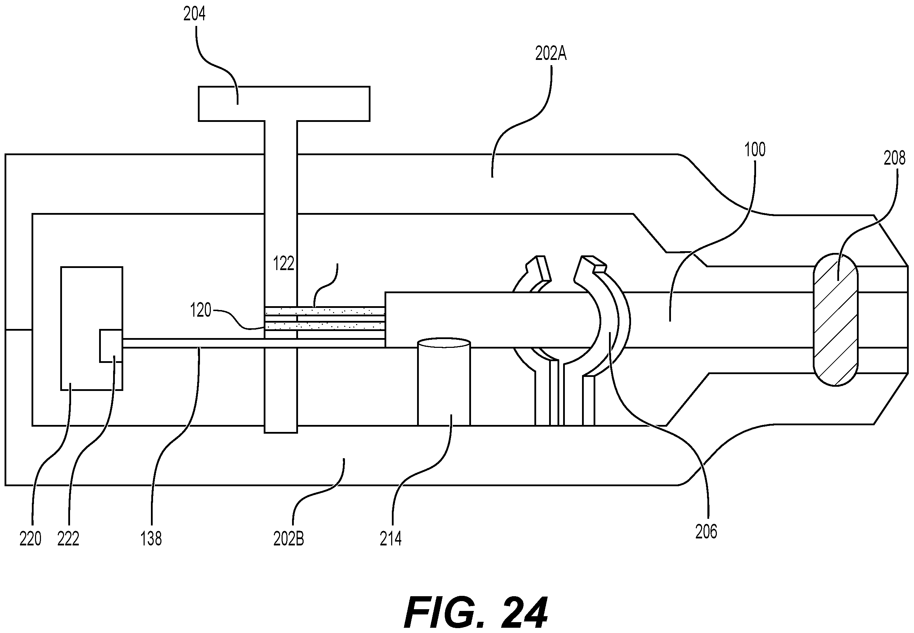

FIG. 22 is a schematic pictorial illustration showing a first connecting feature for connecting a proximal end of the elongated tube 100 with the handle 200 of the medical probe 14. FIG. 23 is a schematic pictorial illustration showing a second connecting feature for connecting the proximal end of the elongated tube 100 with the handle 200 of the medical probe 14. FIG. 24 is a schematic pictorial illustration showing a sectional view of the handle 200.

With specific reference to FIGs. 22-24, and further to the above, the handle housing 202 can be formed from two shells 202A, 202B, to which the pull wire control knob 204 is rotatably connected, with the board connector 220 being disposed in a cavity thereof. The handle 200 and proximal end of the elongated body 100 can be provided with a number of connecting features to simplify the assembly process thereof.

For example, a locating protrusion 214 (formed as, e.g., a pin) can be provided in the handle 200 that aligns with and mates with a complementary locating hole 114 defined in a proximal end of the elongated body 100. Of course, in some examples, this configuration can be flipped such that the hole 114 is defined in the handle 200 and the locating protrusion 214 is formed on the elongated body 100. Moreover, a clip 206 can be provided in the handle 200 that engages the outer wall jacket 106. When used in conjunction with one another, the clip 206 helps to maintain engagement between the locating hole 114 and the locating protrusion 214. Further, the handle 200 can include a seal 208 between the handle 200 and the outer wall 106 of the elongated body 100, which provides a sealing effect without need for adhesive.

The following description assumes, by way of example, that a target region (e.g., a vessel 12A) is to be mapped and/or ablated. Of course, it will be appreciated that the presently described technology may be employed to ablate and/or map other regions of the heart 12.

In an initial step, physician 24 inserts the distal tip 28 is inserted into the subject. The coils 33 and/or the electrodes 26 can be used to navigate the medical probe 14 within the subject. Once the distal tip 28 of the probe 14 is appropriately positioned, the flexible circuit 130, which includes the electrodes 26, is pressed against the vessel 12A.

In a mapping step, once the electrodes 26 are positioned properly in contact with the vessel 12A, the electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

In an ablation step, the physician 24 operates processor 55 and ablation energy generator 50 to supply electrical current to electrodes 26 of the flexible membrane 110. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., patch 38 or the like) coupled to the patient's body.

In some examples, RF sinusoidal-like (alternating) electrical current is supplied to the electrodes 26, such that RF ablation of the tissue is performed. Alternatively, pulsed current (e.g., DC or AC) may be supplied so as to perform irreversible electroporation (IRE) or pulsed field ablation (PFA). When pulsed current is supplied to perform PFA, unipolar ablation energy can be supplied by electrical current running between the electrodes 26 on the flexible membrane 110 and the electrode patch(es) 38 or a backpatch. Moreover, bipolar ablation energy can be supplied by electrical current running between the electrodes 26 on flexible membrane 110 themselves and/or another catheter within another portion of the heart 12.

After the mapping and/or is complete, the physician 24 can retract the distal tip 28 from the vessel 12A and either remove the probe 14 from the subject or more it to another region of the heart 12 in need of mapping and/or ablation.

The disclosed technology described herein can be further understood according to the following clauses:
Clause 1. A medical probe comprising: an elongated body extending along a longitudinal axis from a proximal end to a distal end, the elongated body defining a lumen and comprising: a tube extending along the longitudinal axis, the tube comprising a plurality of slits formed therein, the slits permitting the elongated body to deflect relative to the longitudinal axis; an outer wall jacket surrounding the tube; and a flexible circuit disposed on the outer wall jacket; and one or more electrodes disposed on the flexible circuit and configured to be placed in contact with tissue of an organ.
Clause 2. The medical probe of clause 1, further comprising: one or more coils disposed on the flexible circuit along the longitudinal axis, each coil being configured to generate a current when subj ected to a magnetic field, the current being indicative of a position of the respective coil.
Clause 3. The medical probe of clause 2, each of the coils comprising a generally planar spiral coil.
Clause 4. The medical probe of any one of clauses 1-3, the lumen being defined by the tube.
Clause 5. The medical probe of any one of clauses 1-4, the plurality of slits being formed asymmetrically in the tube.
Clause 6. The medical probe of any one of clauses 1-5, the plurality of slits comprising: a plurality of first slits, each first slit having a first width; and a plurality of second slits, each second slit having a second width that is wider than the first width.
Clause 7. The medical probe of clause 6, each first slit being formed on a first side of the tube and each second slit being formed on a second side of the tube.
Clause 8. The medical probe of any one of clauses 1-7, the elongated body having a first radius of curvature when deflected in a first direction and a second radius of curvature when deflected in a second direction.
Clause 9. The medical probe of any one of clauses 1-8, each slit of the plurality of slits being formed in a direction approximately orthogonal to the longitudinal axis.
Clause 10. The medical probe of any one of clauses 1-9, further comprising a first pull wire, the first pull wire having a first end coupled to a distal end of the elongated body and being configured to move to deflect the elongated body.
Clause 11. The medical probe of clause 10, the first pull wire extending through the lumen along the longitudinal axis.
Clause 12. The medical probe of clause 10, the first pull wire being sandwiched between the tube and the outer wall jacket along the longitudinal axis.
Clause 13. The medical probe of any one of clauses 10-12, further comprising a second pull wire, the second pull wire having a first end coupled to the distal end of the elongated body and being configured to move to deflect the elongated body.
Clause 14. The medical probe of any one of clauses 1-13, the flexible circuit comprising: a first section that extends approximately parallel to the longitudinal axis; one or more second sections that extend from the first section and wrap around the outer wall jacket; and a third section that extends from the first section and wraps around a distalmost end of the elongated body to extend into the lumen.
Clause 15. The medical probe of clause 14, further comprising a long flexible circuit connected to the third section and extending through the lumen.
Clause 16. The medical probe of any one of clauses 14-15, the flexible circuit comprising a single flexible circuit extending near the proximal end of the elongated body to near the distal end of the elongated body.
Clause 17. The medical probe of any one of clauses 1-13, the flexible circuit comprising: a first section that comprises a serpentine shape along the longitudinal axis; and one or more second sections that extend from the first section and at least partially wrap around the outer wall jacket, the one or more electrodes being disposed on at least one of the one or more section sections.
Clause 18. The medical probe of any one of clauses 1-17, further comprising a handle, a proximal end of the elongated body being connected to the handle via a locating protrusion and locating aperture arrangement in which (i) the locating protrusion is disposed on one of the elongated body and handle and (ii) the locating aperture is on the other of the handle and the elongated body.
Clause 19. The medical probe of clause 18, a connector housed within the handle, a proximal end of the long flexible circuit being connected to the connector.
Clause 20. The medical probe of any one of clauses 18-19, the locating hole being defined through a proximal end of the elongated body, and the locating protrusion being provided in the handle and mating with the locating hole along a direction approximately orthogonal to the longitudinal axis.
Clause 21. The medical probe of any one of clauses 18-20, further comprising a clip that engages with the outer wall jacket of the elongated body.
Clause 22. The medical probe of any one of clauses 18-21, further comprising a seal between the handle and the outer wall jacket of the elongated body.
Clause 23. The medical probe of any one of clauses 1-22, the tube being a monolithic member and comprising a biocompatible metal material.
Clause 24. The medical probe of any one of clauses 1-23, the outer wall jacket comprising a biocompatible polymer material.
Clause 25. A method of forming a medical probe, the method comprising the steps of: laser cutting a tube and to a predetermined length; laser cutting a plurality of slits in the tube, the slits permitting the tube to deflect relative to a longitudinal axis; covering an outer surface of the tube with an outer wall jacket; wrapping a flexible circuit at least partially around the outer wall jacket, the flexible circuit comprising one or more electrodes; heating a thermoplastic material over the probe flexible circuit and reflowing the thermoplastic material; and laser drilling an aperture in the thermoplastic material to expose a surface of the one or more electrodes.
Clause 26. The method of clause 25, the probe flexible circuit comprising one or more coils, each coil being configured to generate a current when subjected to a magnetic field, the current being indicative of a position of the respective coil.
Clause 27. The method of any one of clauses 25-26, further comprising the step of: folding the probe flexible circuit to extend a portion of the probe flexible circuit into a lumen of the tube.
Clause 28. The method of clause 27, further comprising the steps of: connecting a distal end of a long flexible circuit to the portion of the probe flexible circuit; and connecting a proximal end of the long flexible circuit to a connector housed within a handle.
Clause 29. The method of any one of clauses 25-29, further comprising the steps of: welding a distal end of a pull wire to a distal tip of the tube; and routing the pull wire along the tube.
Clause 30. The method of clause 29, further comprising the steps of: laser cutting a slot in the tube along the predetermined length of the tube; and positioning the pull wire in the slot.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A medical probe comprising:
an elongated body extending along a longitudinal axis from a proximal end to a distal end, the elongated body defining a lumen and comprising:
a tube extending along the longitudinal axis, the tube comprising a plurality of slits formed therein, the slits permitting the elongated body to deflect relative to the longitudinal axis;
an outer wall jacket surrounding the tube; and
a flexible circuit disposed on the outer wall jacket; and
one or more electrodes disposed on the flexible circuit and configured to be placed in contact with tissue of an organ.

2. The medical probe of claim 1, further comprising:
one or more coils disposed on the flexible circuit along the longitudinal axis, each coil being configured to generate a current when subjected to a magnetic field, the current being indicative of a position of the respective coil.

3. The medical probe of claim 2, each of the coils comprising a generally planar spiral coil.

4. The medical probe of any preceding claim, the lumen being defined by the tube.

5. The medical probe of any preceding claim, the plurality of slits being formed asymmetrically in the tube.

6. The medical probe of any preceding claim, the plurality of slits comprising:
a plurality of first slits, each first slit having a first width; and
a plurality of second slits, each second slit having a second width that is wider than the first width.

7. The medical probe of any preceding claim, the elongated body having a first radius of curvature when deflected in a first direction and a second radius of curvature when deflected in a second direction.

8. The medical probe of any preceding claim, each slit of the plurality of slits being formed in a direction approximately orthogonal to the longitudinal axis.

9. The medical probe of an preceding claim, further comprising a first pull wire, the first pull wire having a first end coupled to a distal end of the elongated body and being configured to move to deflect the elongated body.

10. The medical probe of any preceding claim, the flexible circuit comprising:
a first section that extends approximately parallel to the longitudinal axis;
one or more second sections that extend from the first section and wrap around the outer wall jacket; and
a third section that extends from the first section and wraps around a distalmost end of the elongated body to extend into the lumen.

11. The medical probe of claim 10, further comprising a long flexible circuit connected to the third section and extending through the lumen.

12. The medical probe of claim 10 or claim 11, the flexible circuit comprising a single flexible circuit extending near the proximal end of the elongated body to near the distal end of the elongated body.

13. The medical probe of any preceding claim, the flexible circuit comprising:
a first section that comprises a serpentine shape along the longitudinal axis; and
one or more second sections that extend from the first section and at least partially wrap around the outer wall jacket, the one or more electrodes being disposed on at least one of the one or more section sections.

14. The medical probe of any preceding claim, further comprising a handle, a proximal end of the elongated body being connected to the handle via a locating protrusion and locating aperture arrangement in which (i) the locating protrusion is disposed on one of the elongated body and handle and (ii) the locating aperture is on the other of the handle and the elongated body.

15. The medical probe of claim 14, a locating hole being defined through a proximal end of the elongated body, and the locating protrusion being provided in the handle and mating with the locating hole along a direction approximately orthogonal to the longitudinal axis.

16. The medical probe of claim 14 or claim 15, further comprising a clip that engages with the outer wall jacket of the elongated body.

17. The medical probe of any preceding claim, the tube being a monolithic member and comprising a biocompatible metal material.

18. A method of forming a medical probe, the method comprising the steps of:
laser cutting a tube and to a predetermined length;
laser cutting a plurality of slits in the tube, the slits permitting the tube to deflect relative to a longitudinal axis;
covering an outer surface of the tube with an outer wall jacket;
wrapping a flexible circuit at least partially around the outer wall jacket, the flexible circuit comprising one or more electrodes;
heating a thermoplastic material over the flexible circuit and reflowing the thermoplastic material; and
laser drilling an aperture in the thermoplastic material to expose a surface of the one or more electrodes.

19. The method of claim 18, further comprising the step of:
folding the flexible circuit to extend a portion of the flexible circuit into a lumen of the tube.

20. The method of claim 18 or claim 19, further comprising the steps of:
welding a distal end of a pull wire to a distal tip of the tube; and
routing the pull wire along the tube.
